# EUROPEAN PATENT APPLICATION

(11) **EP 4 548 842 A1**
(43) Date of publication of application: **07.05.2025**
(21) Application number: 23818214.1
(22) Date of filing: 15.09.2023
(51) Int. Cl.: A61B 5/145

(54) **MONITORING APPARATUS AND ELECTRON BEAM IRRADIATION STERILIZATION APPARATUS**

(71) Applicant: Shanghai United Imaging Microelectronics Technology Co., Ltd., Jiading District Shanghai 201800 (CN)
(72) Inventor: LIU, Wei, Shanhai 201800 (CN)
(74) Representative: Viering, Jentschura & Partner mbB Patent- und Rechtsanwälte
(86) International application number: PCT/CN2023/119062
(87) International publication number: WO 2025/054955

(57) **Abstract**

A monitoring device (100) and an electron beam irradiation sterilization device (500) are provided. The monitoring device (100) includes: a main shell (1) provided with a first accommodating chamber (11), a sensing member (2) located in the first accommodating chamber (11), and a sealing cover (3). The sensing member (2) includes a sensor body (21) and a probe (22) connected to the sensor body (21), and is capable of moving relative to the main shell (1). The sealing cover (3) includes a cover body (30) and a groove (31) arranged on the cover body (30), the cover body (30) is connected to the main shell (1) and abuts against the sensor body (21), the groove (31) is defined as a sealing chamber (32) accommodating the probe (22), and the sealing cover (3) forms a radiation shield on the sensor body (21) and expose the probe (22) to electron irradiation.

## Description

### TECHNICAL FIELD

The present invention generally relates to a field of electronic medical technology, and in particular, to a monitoring device and an electron beam irradiation sterilization device.

### BACKGROUND

A blood monitoring device is a wearable device that can continuously monitor blood composition of subcutaneous interstitial fluid. A sensor of the blood monitoring device includes an internal electronic element and an exposed probe, and the sensor is applied to a surface of human skin and implanted into subcutaneous tissue of human body. A user wears the sensor of the blood monitoring device on the surface of the skin, and a blood state is continuously analyzed by the probe of the sensor implanted into the subcutaneous tissue. To meet biosafety requirements of an implanted probe, it is necessary to ensure aseptic safety by sterilization process to prevent local infection caused by bacteria, viruses and other hazards, which pose a threat to health of the user.

At present, a sterilization method for the sensor used in the blood monitoring device mainly includes an ethylene oxide gas sterilization and an electron beam irradiation sterilization. The ethylene oxide gas sterilization is very effective for the electronic element, but ethylene oxide gas would destroy a biological enzyme attached to a surface of the probe, resulting in a failure of the sensor. The electron beam irradiation sterilization can effectively sterilize the probe without destroying the biological enzyme, but an electron beam would cause irreversible damage to the electronic element.

In the related art, the sensor of the blood monitoring device is separated according to a passive part and an active part, the probe in the passive part is sterilized by electron beam irradiation, and the electronic element in the active part is sterilized by ethylene oxide gas. This method can solve incompatibility of sterilization between the passive part and the active part, but it would add additional aseptic packaging, and the passive part and the active part are assembled by additional actions when the user uses the sensor, which increases operation complexity for the user and a risk of inadequate assembly.

### SUMMARY

According to various embodiments of the present invention, a monitoring device and an electron beam irradiation sterilization device are provided.

In a first aspect, the present invention provides a monitoring device, including a main shell, a sensing member, and a sealing cover. The main shell is provided with a first accommodating chamber. The sensing member is located in the first accommodating chamber, the sensing member includes a sensor body and a probe connected to the sensor body, and the sensing member is capable of moving relative to the main shell. The sealing cover includes a cover body and a groove arranged on the cover body, the groove has an opening towards the sensing member, the cover body is connected to the main shell and abuts against the sensor body, the groove is enclosed for the sensing member to define a sealing chamber accommodating the probe, and the sealing cover is configured to form a radiation shield on the sensor body and expose the probe to electron irradiation.

In some embodiments, the cover body is made of a shielding material, and a wall of the groove is made of a non-shielding material.

In some embodiments, the sealing cover is provided with a second accommodating chamber, when a shielding member moves into the second accommodating chamber, the shielding member is capable of being detachably connected to an outer wall of the sealing chamber and shielding the sensor body.

In some embodiments, the sealing chamber is cylindrical.

In some embodiments, the sealing chamber is provided concentrically or eccentrically relative to an axis of the main shell.

In some embodiments, the sealing cover further includes a sealing cover plate and a first side plate. The first side plate is connected to the sealing cover plate, and the second accommodating chamber is defined by the sealing cover plate, an outer wall of the sealing chamber, and the first side plate.

In some embodiments, the sealing cover further includes a second side plate, which is spaced around periphery of the first side plate and connected to the first side plate.

In some embodiments, a connecting member is provided on either or both of an outer wall of the first side plate and an inner wall of the second side plate, a matching member is provided on a side wall of the main shell, and the connecting member is matched and connected to the matching member.

In some embodiments, the connecting member includes at least two guiding members which are spaced apart around either or both of the outer wall of the first side plate and the inner wall of the second side plate, the matching member includes at least two backstop members which are spaced apart around the side wall of the main shell, and the at least two guiding members and the at least two backstop members are matched and connected with each other.

In some embodiments, the monitoring device further includes a fastening ring, an end of the sealing cover away from the sensing member abuts against the fastening ring, and the fastening ring is connected to the main shell, so as to limit the sealing cover.

In some embodiments, a driving member is further provided in the main shell and fixed in the first accommodating chamber, the sensing member is connected to the driving member, and the driving member is capable of pushing the sensing member outside the main shell via the first accommodating chamber.

In some embodiments, the sensor body includes a shell and an electronic element disposed in the shell, the probe is connected to the electronic element and extends out of the shell, and a first sealing member is provided on a connecting location between the shell and the probe.

In some embodiments, the sensing member further includes a steel pin assembly, and the steel pin assembly includes a handle and a steel pin connected to the handle. The handle is provided on an end of the sensor body, the steel pin is sheathed on the probe, and an end of the steel pin away from the handle penetrates through the sensor body and extends to the other end of the sensor body.

In some embodiments, either or both of a second sealing member and a third sealing member are provided, the second sealing member is provided between the handle and the sensor body, and the third sealing member is provided between the groove and the sensor body.

In a second aspect, the present invention further provides an electron beam irradiation sterilization device, including a shielding member and the above monitoring device, a shape of the shielding member is matched to that of the second accommodating chamber, the shielding member is capable of moving into the second accommodating chamber during electron beam irradiation sterilization for shielding the sensor body and exposing the probe to the electron beam.

Details of one or more embodiments of the present invention are set forth in the following accompanying drawings and descriptions. Other features, objectives, and advantages of the present invention become obvious with reference to the description, the accompanying drawings, and the claims.

### BRIEF DESCRIPTION OF THE DRAWINGS

In order to more clearly illustrate the technical solutions in embodiments of the present invention or the conventional art, the accompanying drawings used in the description of the embodiments or the conventional art will be briefly introduced below. It is apparent that, the accompanying drawings in the following description are only some embodiments of the present invention, and other drawings can be obtained by those of ordinary skill in the art from the provided drawings without creative efforts.
FIG. 1 is a schematic diagram of an application scenario of a monitoring device according to some embodiments.
FIG. 2 is a schematic diagram of a monitoring device according to some embodiments.
FIG. 3 is a schematic diagram of a part of a monitoring device according to some embodiments.
FIG. 4 is an exploded schematic diagram of a monitoring device according to other embodiments.
FIG. 5 is a cross-sectional schematic diagram of a monitoring device according to some embodiments.
FIG. 6 is an enlarged schematic diagram of A in FIG. 5.
FIG. 7 is a schematic diagram of a sensing member according to other embodiments.
FIG. 8 is a schematic diagram of a shielding member according to some embodiments.
FIG. 9 is a schematic diagram of a sealing cover under a first view according to some embodiments.
FIG. 10 is a schematic diagram of a sealing cover under a second view according to some embodiments.
FIG. 11 is a schematic diagram of a fastening ring according to some embodiments.
FIG. 12 is a schematic diagram of an electron beam irradiation sterilization device according to some embodiments.
FIG. 13 is a schematic diagram of a process of an electron beam irradiation sterilization according to some embodiments.

In the figures, 100 represents a monitoring device, 1 represents a main shell, 11 represents a first accommodating chamber, 110 represents a chamber port, 12 represents a matching member, 13 represents a driving member, 14 represents a male thread, 2 represents a sensing member, 21 represents a sensor body, 211 represents a shell, 212 represents an electronic element, 213 represents a first sealing member, 22 represents a probe, 3 represents a sealing cover, 30 represents a cover body, 31 represents a groove, 32 represents a sealing chamber, 33 represents a second accommodating chamber, 34 represents a sealing cover plate, 35 represents a first side plate, 36 represents a second side plate, 37 represents a connecting member, 4 represents a fastening ring, 41 represents a female thread, 42 represents a limiting portion, 23 represents a steel pin assembly, 231 represents a handle, 232 represents a steel pin, 5 represents a second sealing member, 6 represents a third sealing member, 200 represents a shielding member, 201 represents a pathway, 300 represent an object to be detected, 400 represents a processing terminal, 500 represents an electron beam irradiation sterilization device, and 600 represents an electron beam.

### DETAILED DESCRIPTION OF THE EMBODIMENT

The technical solutions in embodiments of the present invention will be described clearly and completely below with reference to the accompanying drawings in the embodiments of the present invention. Apparently, the described embodiments are merely some of rather than all of the embodiments of the present invention. All other embodiments acquired by those skilled in the art without creative efforts based on the embodiments of the present invention shall fall within the protection scope of the present invention.

It should be noted that when an assembly is considered to be "arranged on" another assembly, it can be directly arranged on another assembly, or there can be a centered assembly. When an assembly is considered to be "provided on" another assembly, it can be directly provided on another assembly, or there can be a centered assembly at the same time. When an assembly is considered to be "fixed to" another assembly, it can be directly fixed to another assembly, or there can be a centered assembly at the same time.

Unless otherwise defined, all technical and scientific terms used herein have the same meaning as a skilled person in the art would understand. The terminology used in the description of the present invention is for the purpose of describing particular embodiments and is not intended to limit the invention. The term "and/or" as used herein includes any and all combinations of one or more of the associated listed items.

It should be noted that "proximal end" and "proximal side" are used herein to mean an end or a side that is proximal to an operator and in a position or a direction opposite to the terms "distal end" and "distal side", and "distal end" and "distal side" refer to an end or a side that is far away from the operator and in a position or a direction opposite to the terms "proximal end" and "proximal side".

In recent years, micro destructive, non-invasive, and continuous blood glucose monitoring devices have emerged and evolved towards miniaturization, functional diversity, intelligence, and measurement accuracy, bringing revolutionary advances in monitoring blood glucose levels of an object to be detected. Physical condition of the object to be detected can be known by the blood glucose monitoring devices in order to provide a real-time and accurate response.

A continuous monitoring device can apply a sensor to a surface of human skin and implant a probe of the sensor into subcutaneous tissue of human body, the probe can continuously analyze blood status information, such as glucose-related data, neutral lipid-related data, blood oxygen parameters, etc. To meet biosafety requirements of an implanted probe, it is necessary to ensure aseptic safety by sterilization process to prevent local infection caused by bacteria, viruses and other hazards, which pose a threat to health of the user.

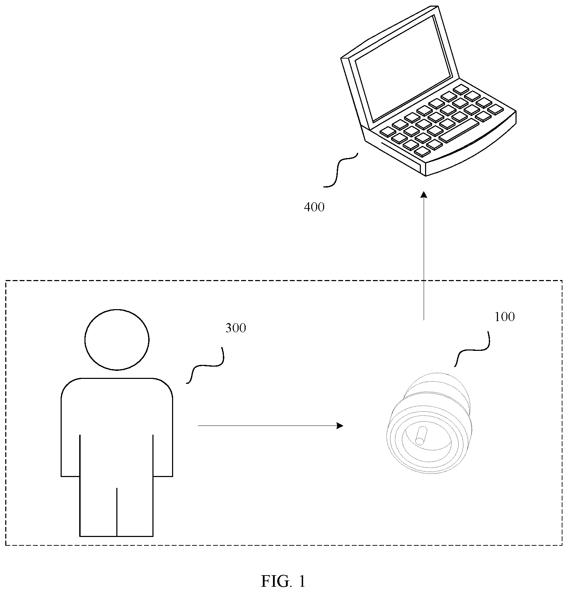

The following proceeds with a detailed description in conjunction with accompanying drawings. FIG. 1 is a schematic diagram of an application scenario of a monitoring device 100 provided in the present embodiment. Referring to FIG. 1, the monitoring device 100 may push a sensor to a skin surface of an object 300 to be detected, acquire blood data of the object 300 to be detected by a probe of the sensor, and send the blood data to a processing terminal 400 by a network. The processing terminal 400 may receive the blood data of the object 300 to be detected, and give relevant guidance information or suggestions after analyzing the blood data, so as to facilitate control of the blood state. The processing terminal 400 may be a cell phone, a tablet, or a computer, etc., and the processing terminal 400 may be installed with corresponding monitoring software.

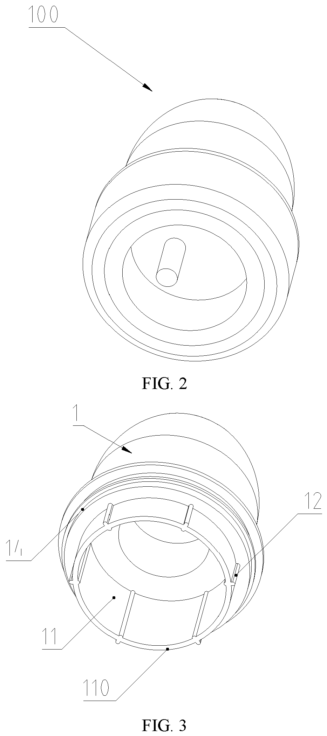

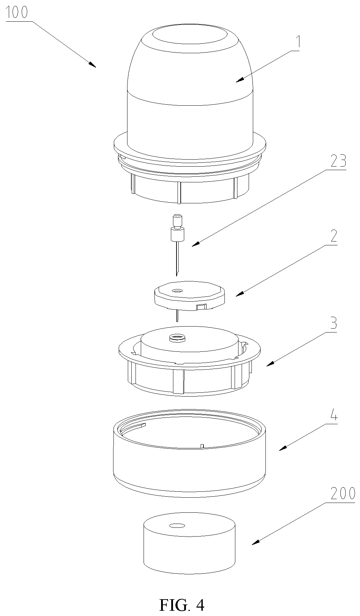

Referring to FIG. 2 and FIG. 4, the present invention provides a monitoring device 100, applied to monitor the blood status of the object 300 to be detected in a field of electronic medical technology. The monitoring device 100 includes a main shell 1, a sensing member 2, and a sealing cover 3.

The main shell 1 is provided with a first accommodating chamber 11, which has a chamber port 110. The sensing member 2 is located in the first accommodating chamber 11.

In the present embodiment, the main shell 1 may be cylindrical and have a circular cross-section, or be in a shape of a circular plate or other irregular shape. The main shell 1 may be made of materials such as PE (polyethylene), PP (polypropylene), PS (polystyrene), PVC (polyvinyl chloride), or PET (polyethylene glycol terephthalate). In some embodiments, the main shell 1 may be integrally formed. In some embodiments, the main shell 1 may be provided in segments, a plurality of segments may be fixed by welding. In other embodiments, the plurality of segments may also be fixed by other fixing means such as screwing, hinging, or clamping.

Referring to FIG. 4, FIG. 5, and FIG. 7, the sensing member 2 includes a sensor body 21 and a probe 22 connected to the sensor body 21, and the sensing member 2 is capable of moving relative to the main shell 1. Specifically, in an initial state, the sensor body 21 and the probe 22 may be accommodated in the main shell 1. In a use state, the sensor body 21 and the probe 22 may be detached from initial positions and move relative to the main shell 1 by an external force, and the probe 22 may move outside the main shell 1 and be implanted in a body surface to detect relevant data in the blood.

In some embodiments, the sensor body 21 may include a shell 211 and an electronic element 212 disposed in the shell 211, and the probe 22 may be connected to the electronic element 212 and extend out of the shell 211. In some embodiments, the electronic element 212 may include a microprocessor, a power management module, an acquisition and amplification module, an A/D (analog to digital) converting module, a wireless communication module, and a battery. The wireless communication module may enable communication between the processing terminal 400 and the sensor body 21 in a non-contact communication manner.

A first sealing member 213 is provided on a connecting location between the shell 211 and the probe 22. The first sealing member 213 may fill a gap between the probe 22 and the shell 211, so as to form an airtight space inside the sensor body 21. The first sealing member 213 may be an O-ring, a silicone, a UV light solid glue, and the like. The first sealing member 213 may be disposed at the connecting location between the shell 211 and the probe 22, and a physical isolation from an external environment may be defined, so as to effectively prevent gases and liquids in the external environment from entering into an interior of the sensing member 2, and improve precision and stability of the sensing member 2.

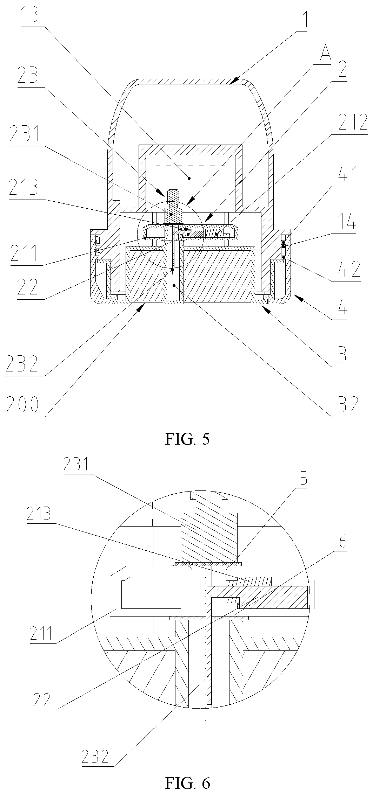

Referring to FIG. 5 and FIG. 6, in some embodiments, in order to ensure that the probe 22 of the sensing member 2 is smoothly implanted into the body surface and is not damaged during an implantation process, the sensing member 2 may further include a steel pin assembly 23. The steel pin assembly 23 may include a handle 231 and a steel pin 232 connected to the handle 231.

The handle 231 may be disposed at an end of the sensor body 21, so that the steel pin 232 may be connected and fixed to the sensor body 21 by the handle 231.

The steel pin 232 may be sheathed on the probe 22, and an end of the steel pin 232 away from the handle 231 may penetrate through the sensor body 21 and extend to the other end of the sensor body 21. In the use state, when the sensor body 21 moves relative to the main shell 1, the steel pin assembly 23 may move in synchronization with the probe 22 relative to the main shell 1. This may protect an exposed probe 22 of the sensing member 2 and aid in the implantation of the probe 22 into the body surface. When the steel pin 232 and the probe 22 are synchronized to complete the implantation in the body surface, the steel pin 232 may be subsequently withdrawn manually or automatically to achieve withdrawal of pin, leaving the probe 22 in the subcutaneous tissue in the body surface.

To meet biosafety requirements of the implanted probe 22, it is necessary to ensure aseptic safety by sterilization process before the probe 22 is implanted and to prevent local infection caused by bacteria, viruses, and other hazards that pose a threat to health of the user.

In the related art, the sensor of the monitoring device is separated according to a passive part and an active part, the probe in the passive part is sterilized by electron beam irradiation, and the electronic element in the active part is sterilized by ethylene oxide gas. This method can solve incompatibility of sterilization between the passive part and the active part, but it would add additional aseptic packaging, and the passive part and the active part are assembled by additional actions when the user uses the sensor, which increases operation complexity for the user and a risk of inadequate assembly.

In order to ensure that the electron beam irradiation sterilization of the probe 22 is completed when product structure is intact and to avoid additional product assembly processes, the monitoring device 100 is further provided with a sealing cover 3. This is described in detail below in conjunction with the accompanying drawings.

Referring to FIG. 3 to FIG. 5 and FIG. 9, the monitoring device 100 is further provided with a sealing cover 3 sealing off the chamber port 110 of the main shell 1. Specifically, the sealing cover 3 includes a cover body 30 and a groove 31 arranged on the cover body 30, the groove 31 has an opening towards the sensing member 2, the cover body 30 is connected to the main shell 1 and abuts against the sensor body 21, and the groove 31 is enclosed for the sensing member 2 to define a sealing chamber 32 accommodating the probe 22.

In some embodiments, an upper end surface of the groove 31 of the sealing cover 3 may abut against a lower surface of the sensing member 2, the probe 22 may extend out of the shell 211 of the sensing member 2 and be accommodated in the groove 31, the groove 31 may be enclosed for the shell 211 to define the sealing chamber 32 for accommodating the probe 22, and the sealing chamber 32 may serve as a physically isolated region for the probe 22.

In other embodiments, referring to FIG. 5, the sensing member 2 may further include a steel pin assembly 23, the steel pin assembly 23 may include a handle 231 and a steel pin 232 connected to the handle 231. The handle 231 may be provided on an end of the sensor body 21, the steel pin 232 may be sheathed on the probe 22, and an end of the steel pin 232 away from the handle 231 may penetrate through the sensor body 21 and extend to the other end of the sensor body 21. At this moment, the upper end surface of the groove 31 of the sealing cover 3 may abut against the lower surface of the sensing member 2, the handle 231 may be provided on the end of the sensor body 21, the steel pin 232 connected to the handle 231 and the probe 22 may extend out of the shell 211 of the sensing member 2 and be accommodated in the groove 31, and the groove 31 may be enclosed for the shell 211 and the handle to define a sealing chamber 32 for accommodating the probe 22.

The sealing cover 3 is configured to form a radiation shield on the sensor body 21 and expose the probe 22 to electronic irradiation.

In some embodiments, the cover 30 may be made of a shielding material and a wall of the groove 31 may be made of a non-shielding material. It may be appreciated that when the monitoring device 100 needs irradiation sterilization by an electron beam 600, the cover body 30 may abut against the sensor body 21 along at least one irradiation direction of the electron beam, since the cover body 30 is disposed relative to the sensor body 21, the cover body 30 may shield electronic irradiation and avoid irreversible damage to the electronic element in the sensor body 21. In addition, the groove 31 may be made of the non-shielding material, so that the probe 22 may be exposed to the electronic irradiation for irradiation sterilization, resulting in a sterile environment in the sealing chamber 32. Alternatively, the shielding material may be any material capable of blocking (or substantially blocking) radiation transmission. The shielding material may be metals such as lead, aluminum, alloy, and other stainless steel materials, or high-density polymers, which are not limited by the present invention.

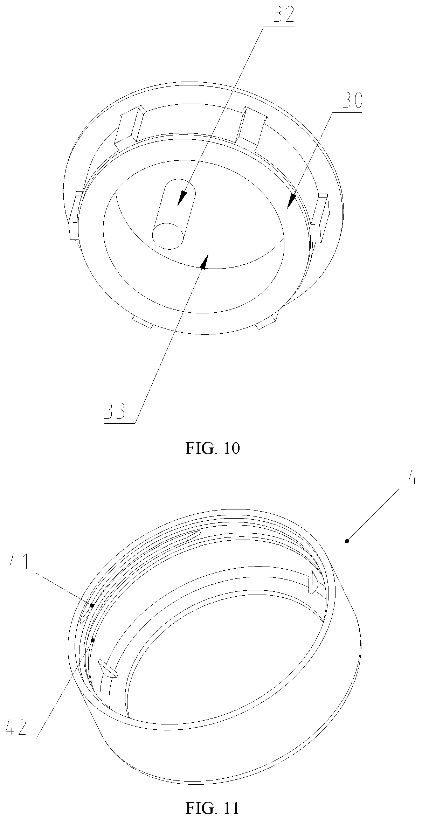

In some embodiments, referring to FIG. 10, the sealing cover 3 may be provided with a second accommodating chamber 33, the second accommodating chamber 33 may be available for a shielding member 200 to move into or out, and the shielding member 200 is capable of being detachably connected to an outer wall of the sealing chamber 32 and shielding the sensor body 21 when the shielding member moves into the second accommodating chamber 33. Alternatively, a detachable connection may include sheathing, clamping, threaded connection, etc., which are not limited in the present invention.

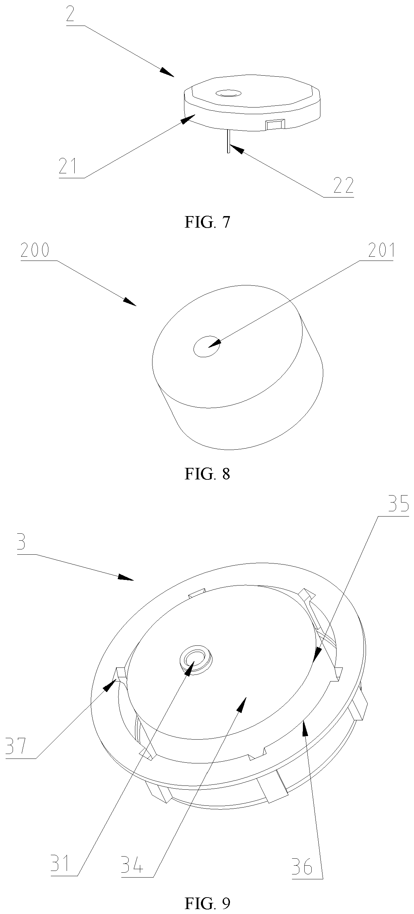

Referring to FIG. 8, in the present embodiment, the shielding member 200 may be made of any material capable of blocking (or substantially blocking) radiation transmission. The shielding member 200 may be made of metals such as lead, aluminum, alloy, and other stainless steel materials, or high density polymers, which are not limited in the present invention. A shape of the shielding member 200 may be matched to that of the second accommodating chamber 33. Alternatively, the shielding member 200 may be provided with a pathway 201 that allows the electron beam 600 to pass through. A shape of the pathway 201 may be cylindrical, conical, variable cross-section, etc., and the shape of the pathway 201 may be matched to that of the second accommodating chamber 33. When the shield member 200 is sheathed to the outer wall of the sealing chamber 32, the sealing chamber 32 may pass through the pathway 201. In this way, when the shield member 200 moves into the second accommodating chamber 33 of the sealing cover 3, the shield member 200 may shield the sensor body 21 and expose the probe 22 to the electron beam 600.

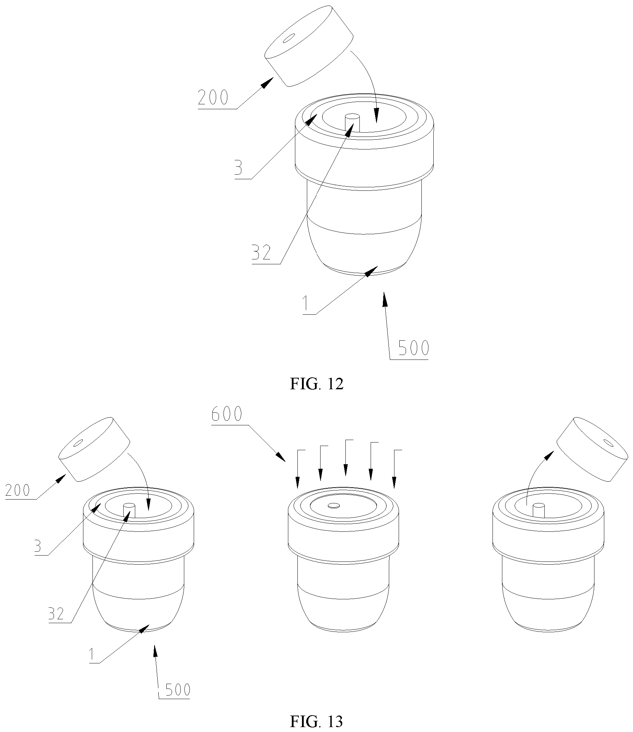

Referring to FIG. 13, when the monitoring device 100 needs irradiation sterilization by the electron beam 600, the shielding member 200 may move into the second accommodating chamber 33 of the sealing cover 3. When the shielding member 200 moves into the second accommodating chamber 33 of the sealing cover 3, the shielding member 200 may be sheathed on the outer wall of the sealing chamber 32 and the sealing chamber 32 may pass through the pathway 201, so that the shielding member 200 may shield the sensor body 21 to form a shielding region, preventing the electronic element 212 in the sensor body 21 from being irradiated by the electron beam 600 and damaged. Meanwhile, the probe 22 may be exposed to the electron beam 600 to form a non-shielding region, and the electron beam 600 may pass through the pathway 201 of the shielding member 200 and the sealing chamber 32 to perform irradiation sterilization to the probe 22 and the steel pin 232 by the electron beam 600, so as to form a sterile environment in the sealing chamber 32.

When the irradiation sterilization of the electron beam 600 is complete, the shielding member 200 may be removed, and a one-piece irradiation sterilization of the electron beam 600 for the monitoring device 100 may be completed.

In some embodiments, the sealing chamber 32 may be cylindrical, and the sealing chamber 32 is provided concentrically or eccentrically relative to an axis of the main shell 1. When the sealing chamber 32 is provided eccentrically relative to the axis of the main shell 1, the sealing chamber 32 may pass through the pathway 201 on the shielding member 200 when the shielding member 200 moves into or out of the second accommodating chamber 33, such that the shielding member 200 can only move axially relative to the monitoring device 100 along a direction that the shielding member 200 moves into or out, and cannot rotate along a radial direction of the sealing cover 3 relative to the sealing cover 3. In this way, the shielding member 200 may be limited in the second accommodating chamber 33, without a need to additionally set up a limiting mechanism, which reduces the processing cost, and the shielding member 200 may cooperate with the sealing cover 3 in a more solid manner when sterilized. In other embodiments, the sealing chamber 32 may be square or in other irregular shapes, as long as the shape of the sealing chamber 32 is matched to that of the shielding member 200 to penetrate through the shielding member 200, which is not limited in the present invention.

Referring to FIG. 9 to FIG. 10, the sealing cover 3, as a part of the product structure of the monitoring device, in an aspect, may protect the probe 22 to form a physically isolated sterile space around the probe 22, and in another aspect, may serve as a placeholder to accommodate the shielding member 200. In other words, the sealing cover 3 may be used for two purposes.

In some embodiments, the sealing cover 3 may further include a sealing cover plate 34 and a first side plate 35.

The sealing cover plate 34 may be provided with the groove 31. Specifically, the sealing cover plate 34 may include a first surface relatively proximal to the sensing member 2 and a second surface relatively far away from the sensing member 2, the first surface may be provided with the groove 31 that has an opening towards the sensing member 2, and the groove 31 may be sealed and connected to the sensor body 21. Alternatively, a third sealing member 6 may be provided between the groove 31 and the sensor body 21. The third sealing member 6 may improve sealing effect of the sealing chamber 32 and ensure sealing performance of the sealing chamber 32. The third sealing member 6 may be integrally formed into the sealing chamber 32 or the sensor body 21, i.e., the third sealing member 6 may be configured as a part of the sealing chamber 32 or as a part of the shell 211 of the sensor body 21. The third sealing member 6 may also be an O-ring, a silicone, a UV light solid glue, etc.

Furthermore, the sealing cover 3 may be provided with a second accommodating chamber 33, the second accommodating chamber 33 may allow the shielding member 200 to move in or out. In some embodiments, the first side plate 35 may be connected to the sealing cover plate 34, and the second accommodating chamber 33 may be defined by the sealing cover plate 34, the outer wall of the sealing chamber 32, and the first side plate 35. Specifically, the second accommodating chamber 33 may be defined by the second surface of the sealing cover plate 34, the outer wall of the sealing chamber 32, and the inner side wall of the first side plate 35. The second accommodating chamber 33 may be roughly cylindrical, cylindrical, or in other shapes, as long as the shape of the second accommodating chamber 33 is matched to that of the shielding member 200, which is not limited in the present invention.

The monitoring device 100 may further include a limiting structure. In some embodiments, the sealing cover 3 may further include a second side plate 36, which is spaced around periphery of the first side plate 35 and connected to the first side plate 35. A connecting member 37 may be provided on either or both of an outer wall of the first side plate 35 and an inner wall of the second side plate 36, a matching member 12 may be provided on a side wall of the main shell 1, and the connecting member 37 may be matched and connected to the matching member 12. In this way, it may avoid rotational movement of the sealing cover 3 relative to the main shell when the sealing cover 3 is connected to the main shell 1, and the connection between the sealing cover 3 and the main shell 1 may be more secure.

In some embodiments, the connection member 37 may be provided as a guiding member recessed in either or both of the outer wall of the first side plate 35 and the inner wall of the second side plate 36, and the matching member 12 may be provided as a backstop member recessed in the side wall of the main shell 1. Alternatively, in some embodiments, the guiding member may include a guiding groove and the corresponding backstop member may include a guiding convex rib. The match between the guiding member and the backstop member may be used as a guide to guide the sealing cover 3 to mount on the main shell 1, thereby providing precise positioning of the sealing cover 3.

In some embodiments, at least two guiding members may be spaced apart along a circumferential direction of either or both of the outer wall of the first side plate 35 and the inner wall of the second side plate 36, and at least two backstop members may be spaced apart around the side wall of the main shell 1 correspondingly. Alternatively, the guiding members and the backstop members may be provided in two, four, six, or other numbers, respectively.

Correspondingly, in some embodiments, at least two guiding pillars 12 may be spaced apart around the side wall of the main shell 1, and the at least two guide pillars 12 may be correspondingly clamped to at least two guiding grooves 37. In this way, when the sealing cover 3 is matched and connected to the main shell 1, the at least two guiding grooves 37 provided on either or both of the outer wall of the first side plate 35 and the inner wall of the second side plate 36 may be clamped and fixed to the at least two guiding pillars 12 provided on the side wall of the main shell 1 correspondingly. In some embodiments, each of the guiding grooves may extend along an axis of the sealing cover 3. In this way, a relative rotational movement between the sealing cover 3 and the main shell 1 may be limited, so that the sealing cover 3 may only be translated up and down along an axial direction of the main shell 1, ensuring that the steel pin 232 and the probe 22 are always in an axial direction of the sealing chamber 32.

Referring to FIG. 11 and FIG. 4, in some embodiments, the monitoring device 100 may further include a fastening ring 4, an end of the sealing cover 3 away from the sensing member 2 may abut against the fastening ring 4, and the fastening ring 4 may be connected to the main shell 1, so as to limit the sealing cover 3.

In some embodiments, the fastening ring 4 may be provided with a female thread 41, the side wall of the main shell 1 may be further provided with a male thread 14, and the fastening ring 4 may be threadedly connected to the main shell 1, thereby limiting an axial movement of the fastening ring 4 relative to the main shell. Specifically, the end of the sealing cover 3 away from the sensing member 2 may abut against the fastening ring 4, and the fastening ring 4 may threadedly connected to the main shell by the male thread 14 of the side wall of the main shell and the female thread 41. The fastening ring 4 is configured to press and fix the sealing cover 3 to further limit an axial movement of the sealing cover 3 relative to the main shell 1.

In order not to interfere with the at least two matching members 12 on the side wall of the main shell 1 and to ensure that the fastening ring 4 presses and fixes the sealing cover 3, the matching members 12 on the side wall of the main shell 1 may be spaced apart from the male thread 14 along the axis of the main shell 1.

In other embodiments, in addition to a threaded match manner between the fastening ring 4 and the main shell 1, the fastening ring 4 and the main shell 1 may also be matched and connected by a clamping fit, an interference fit, or the like, and a matching motion of the fastening ring 4 and the main shell 1 may be either circumferential rotation or axial buckling, which is not limited in the present invention.

In some embodiments, the fastening ring 4 may be further provided with a limiting portion 42, the limiting portion 42 may be configured to be matched and connected to the sealing cover 3. Since the limiting portion 42 is matched and connected to the sealing cover 3, the limiting portion 42 may further limit movement of the sealing cover 3 relative to the fastening ring 4 along the axis of the main shell 1.

In some embodiments, a driving member 13 may be further provided in the main shell 1 and fixed in the first accommodating chamber 11, the sensing member 2 may be connected to the driving member 13, and the driving member 13 is capable of pushing the sensing member 2 outside the main shell 1 via the chamber port 110 of the first accommodating chamber 11.

In some embodiments, the driving member 13 may include an ejection mechanism, and the driving member 13 may include an elastic member. In an initial state, the elastic member may be in an empowered state, and in a use state, the elastic member may undergo elastic deformation and drive the sensing member 2 to move. In other embodiments, the driving member 13 may be not limited to the above, as long as the driving member 13 can drive the sensing member 2 to move relative to the main shell 1, and the present invention will not repeat herein.

Alternatively, the monitoring device 100 may further include a trigger member provided on the main shell 1, and the trigger member may be connected to the drive member 13 for triggering and actuating the drive member 13. When the trigger member is triggered by an external force, the drive member 13 may actuate the sensing member 2 to move relative to the main shell 1 from a proximal end to a distal end until the sensing member 2 is affixed to a skin surface, and the probe 22 may be implanted into the subcutaneous tissue of the body surface.

Referring to FIG. 6, in some embodiments, a second sealing member 5 may be provided between the handle 231 and the sensor body 21. In some embodiments, a third sealing member 6 may be provided between the groove 31 and the sensor body 21. The second sealing member 5 and the third sealing member 6 may be an O-ring, a silicone, a UV light solid glue, and the like.

Referring to FIG. 12, the present invention further provides an electron beam irradiation sterilization device 500, including a shielding member 200 and a monitoring device 100 as described above.

The shape of the shielding member 200 may be matched to that of the second accommodating chamber 33, and the shielding member 200 is capable of moving into the second accommodating chamber 33 during irradiation sterilization of the electron beam 600 for shielding the sensor body 21 and exposing the probe 22 to the electron beam 600.

In the present embodiment, the shielding member 200 may be not a part of the monitoring device 100, and the shielding member 200 may move into the second accommodating chamber 33 of the sealing cover 3 and be sheathed on the outer wall of the sealing chamber 32. The shielding member 200 may shield the sensor body 21 to form a shielding region to protect the sensor body 21 from irradiation damage by the electron beam 600. Meanwhile, the probe 22 may be exposed to the electron beam 600 to form a non-shielding region, and the electron beam 600 may vertically pass through the pathway 201 of the shield member 200 and the sealing chamber 32 to perform irradiation sterilization on the probe 22 by the electron beam 600. After the irradiation sterilization by the electron beam 600 is completed, the shielding member 200 may be removed, and the monitoring device may be labeled and packed into a box for sale.

The various technical features of the above-described embodiments may be combined in any combination, and for the sake of brevity of description, all possible combinations of the various technical features of the above-described embodiments have not been described, however, as long as there is no contradiction in the combinations of these technical features, these should all be considered to be within the scope of the present description as documented herein.

One skilled in the art should realize that the above embodiments are only used to illustrate the present invention, and are not intended to be used as a limitation of the present invention, and as long as they are within the scope of the spirit of the substance of the present invention, appropriate alterations and variations of the above embodiments fall within the scope of the claimed protection of the present invention.

## Claims

1. A monitoring device, **characterized by** comprising a main shell (1), a sensing member (2), and a sealing cover (3),
wherein the main shell (1) is provided with a first accommodating chamber (11);
the sensing member (2) is located in the first accommodating chamber (11), the sensing member (2) comprises a sensor body (21) and a probe (22) connected to the sensor body (21), and the sensing member (2) is capable of moving relative to the main shell (1); and
the sealing cover (3) comprises a cover body (30) and a groove (31) arranged on the cover body (30), the groove (31) has an opening towards the sensing member (2), the cover body (30) is connected to the main shell (1) and abuts against the sensor body (21), the groove (31) is enclosed for the sensing member (2) to define a sealing chamber (32) accommodating the probe (22), and the sealing cover (3) is configured to form a radiation shield on the sensor body (21) and expose the probe (22) to electron irradiation.

2. The monitoring device of claim 1, wherein the cover body (30) is made of a shielding material, and a wall of the groove (31) is made of a non-shielding material.

3. The monitoring device of claim 1, wherein the sealing cover (3) is provided with a second accommodating chamber (33), when a shielding member (200) moves into the second accommodating chamber (33), the shielding member (200) is capable of being detachably connected to an outer wall of the sealing chamber (32) and shielding the sensor body (21).

4. The monitoring device of any one of claims 1 to 3, wherein the sealing chamber (32) is cylindrical.

5. The monitoring device of any one of claims 1 to 4, wherein the sealing chamber (32) is provided concentrically or eccentrically relative to an axis of the main shell (1).

6. The monitoring device of any one of claims 1 to 5, wherein the sealing cover further comprises a sealing cover plate (34) and a first side plate (35); and
the first side plate (35) is connected to the sealing cover plate (34), and the second accommodating chamber (33) is defined by the sealing cover plate (34), an outer wall of the sealing chamber (32), and the first side plate (35).

7. The monitoring device of claim 6, wherein the sealing cover (3) further comprises a second side plate (36), which is spaced around periphery of the first side plate (35) and connected to the first side plate (35).

8. The monitoring device of claim 7, wherein a connecting member (37) is provided on either or both of an outer wall of the first side plate (35) and an inner wall of the second side plate (36), a matching member (12) is provided on a side wall of the main shell (1), and the connecting member (37) is matched and connected to the matching member (12).

9. The monitoring device of claim 8, wherein the connecting member (37) comprises at least two guiding members which are spaced apart around either or both of the outer wall of the first side plate (35) and the inner wall of the second side plate (36),
the matching member (12) comprises at least two backstop members which are spaced apart around the side wall of the main shell (1), and
the at least two guiding members and the at least two backstop members are matched and connected with each other.

10. The monitoring device of any one of claims 1 to 9, further comprising a fastening ring (4), wherein an end of the sealing cover (3) away from the sensing member (2) abuts against the fastening ring (4), and the fastening ring (4) is connected to the main shell (1), so as to limit the sealing cover (3).

11. The monitoring device of any one of claims 1 to 10, wherein a driving member (13) is further provided in the main shell (1) and fixed in the first accommodating chamber (11), the sensing member (2) is connected to the driving member (13), and the driving member (13) is capable of pushing the sensing member (2) outside the main shell (1) via the first accommodating chamber (11).

12. The monitoring device of any one of claims 1 to 11, wherein the sensor body (21) comprises a shell (211) and an electronic element (212) disposed in the shell (211), the probe (22) is connected to the electronic element (212) and extends out of the shell (211), and a first sealing member (213) is provided on a connecting location between the shell (211) and the probe (22).

13. The monitoring device of claim any one of claims 1 to 12, wherein the sensing member (2) further comprises a steel pin assembly (23), and the steel pin assembly (23) comprises a handle (231) and a steel pin (232) connected to the handle (231);
the handle (231) is provided on an end of the sensor body (21); and
the steel pin (232) is sheathed on the probe (22), and an end of the steel pin (232) away from the handle (231) penetrates through the sensor body (21) and extends to the other end of the sensor body (21).

14. The monitoring device of claim 13, wherein either or both of a second sealing member (5) and a third sealing member (6) are provided, the second sealing member (5) is provided between the handle (231) and the sensor body (21), and the third sealing member (6) is provided between the groove (31) and the sensor body (21).

15. An electron beam irradiation sterilization device, **characterized by** comprising a shielding member (200) and the monitoring device (100) of any one of claims 1 to 14, wherein a shape of the shielding member (200) is matched to that of the second accommodating chamber (33), the shielding member (200) is capable of moving into the second accommodating chamber (33) during electron beam irradiation sterilization for shielding the sensor body (21) and exposing the probe (22) to the electron beam.
